# EUROPEAN PATENT SPECIFICATION

(11) **EP 3 320 890 B1**
(45) Date of publication and mention of the grant of the patent: **26.02.2020**
(21) Application number: 16821471.6
(22) Date of filing: 07.07.2016
(51) Int. Cl.: A61K 8/39, A61K 8/46, A61Q 5/02, C11D 1/18, C11D 1/74

(54) **HAIR SHAMPOO COMPOSITION**
HAARSHAMPOOZUSAMMENSETZUNG
COMPOSITION DE SHAMPOOING POUR LES CHEVEUX

(30) Priority: 09.07.2015 JP 2015137395
(43) Date of publication of application: 16.05.2018
(73) Proprietor: NOF Corporation, Shibuya-ku Tokyo 150-6019 (JP)
(72) Inventor: SHIMADA, Masahiko, Amagasaki-shi Hyogo 660-0095 (JP)
(74) Representative: Williams Powell
(86) International application number: PCT/JP2016/070176
(87) International publication number: WO 2017/007003

(56) References cited:
- WO-A1-2005/078039
- JP-A- 2000 109 888
- JP-A- 2001 181 675
- JP-A- 2004 323 474
- JP-A- 2008 013 513
- US-A- 6 165 451

## Description

### [Technical Field]

The present invention relates to a hair shampoo composition.

### [Background Art]

Generally, hair shampoo compositions (hereinafter, also referred to as hair shampoos, shampoos, or preparations) comprise, as essential components, an anionic surfactant, which is a washing and foaming agent, an amphoteric surfactant for improving the stability and the foam quality of the preparation, a thickener, which provides a suitable degree of viscosity to the preparation, a cationic polymer for improving finger combability when rinsing, and the like. Hair shampoo is a preparation which removes dirt from the hair and the scalp and keeps the hair and the scalp healthy, but when the hair and the scalp are dried with a dryer or the like after shampooing, the moisture of the scalp is sometimes diminished due to excessive degreasing caused as a result of use of the hair shampoo or the like. That is to say, using hair shampoo sometimes actually causes discomfort to a user, and in extreme cases has led to skin problems on the scalp.

It has been proposed to add specific additives to anionic surfactants and amphoteric surfactants for the purpose of suppressing the drying of the scalp after shampooing. For example, in Patent Literature 1, a liquid cleanser composition is proposed, in which an acyl glutamate, an acyl aspartate, an amphoteric surfactant, and a sugar alcohol are blended.

Further, in addition to suppressing drying, Patent Literature 2 proposes, for the purpose of giving a suitable viscosity to the preparation, a cleanser composition obtained by blending an alkyl amidopropyl betaine, a sodium alkyl ether sulfate or a sodium acyl methyl taurate, an alkyl methyl monoethanolamide and a polyhydric alcohol; furthermore, Patent Literature 3 proposes a skin cleanser composition obtained by blending an alkyl ether sulfuric acid ester salt, a betaine type amphoteric surfactant, an alkylamine oxide, a polyether-modified silicone and a gloss imparting agent.

However, the cleanser compositions described above have not yet been satisfactory in terms of drying of the scalp after shampooing.

The acyl glutamates and acyl aspartates blended in the liquid cleanser composition of Patent Literature 1 and the sodium acyl methyl taurate blended in the cleanser composition of Patent Literature 2 are known to be relatively mild on the scalp, but even when these have been blended, satisfactory effects have not been obtained.

Further, the alkyl methyl monoethanolamide blended in the cleanser composition of Patent Literature 2, and the alkyl amine oxide blended in the skin cleanser composition of Patent Literature 3 are components that produce the effect of giving a suitable viscosity to the preparation, but they have led to unfavorable effects in terms of drying of the scalp after shampooing. As described above, however, thickeners such as alkyl methyl monoethanolamides and alkyl amine oxides are indispensable components for the functions required for hair shampoo compositions and for stabilizing the preparations, and thus technical problems have remained.

### [Citation List]

### [Patent Literature]

[PTL 1] Japanese Unexamined Patent Application Publication No. 2014-5230
[PTL 2] Japanese Patent No. 5667790
[PTL 3] Japanese Unexamined Patent Application Publication No. 2014-76975

### [Summary of Invention]

### [Technical Problem]

An object of the present invention is to provide a hair shampoo composition with which the preparation maintains a viscosity suitable for use, and which does not diminish the moisture of the scalp following shampooing, even after further drying with a dryer immediately following towel drying.

### [Solution to Problem]

The present inventors discovered that the object described above can be achieved by combining a taurine salt of an N-acyl methyl taurate represented by the following general formula (1) with an alkylene oxide derivative represented by the following general formula (2).

That is to say, the present invention provides hair shampoo composition comprising the following components (A) and (B):
(A) a taurine salt of an N-acyl methyl taurate represented by the following general formula (1) at 0.5 to 15 mass%

   R¹CON(CH₃)CH₂CH₂SO₃⁻X⁺ (1)

   (wherein R¹CO is an acyl group having 8 to 24 carbon atoms, X is H₃N⁺CH₂CH₂SO₃⁻M⁺, and M is an alkali metal or organic ammonium)
(B) an alkylene oxide derivative represented by the following general formula (2) at 0.3 to 10 mass%

   R²COO(BO)ₘH (2)

   (wherein R²CO is an acyl group having 8 to 14 carbon atoms, BO is an oxybutylene group, m is the average addition molar number, and 1 ≤ m ≤ 2),
   wherein the mass ratio of component (A) to component (B) is (A)/(B) = 0.5 to 8.

The hair shampoo composition of the present invention may further comprise component (C):
(C) an alkylene oxide derivative represented by the following general formula (3)

R³COO(BO)ₙOCR⁴ (3)

(wherein R³CO and R⁴CO are each independently an acyl group having 8 to 14 carbon atoms, BO is an oxybutylene group, n is the average addition molar number, and 1 ≤ n ≤ 2).

### [Advantageous Effects of Invention]

According to the present invention, it is possible to produce effects such that the preparation maintains a viscosity suitable for use, and does not diminish the moisture of the scalp following shampooing, even after further drying with a dryer immediately following towel drying.

### [Description of Embodiments]

Hereafter, the present invention will be described in detail.

The hair shampoo composition of the present invention at least comprises the following components (A) and (B).

### Component (A)

Component (A) is a taurine salt of an N-acyl methyl taurate represented by the following general formula (1).

R¹CON(CH₃)CH₂CH₂SO₃⁻X⁺ (1)

In the formula, R¹CO is an acyl group having 8 to 24 carbon atoms, and preferably 8 to 18 carbon atoms, and specific examples of such an acyl group include a capryloyl group, a caproyl group, a lauroyl group, a myristoyl group, a palmitoyl group, a stearoyl group, an oleoyl group and a behenoyl group. Furthermore, mixed acyl groups including two or more of these acyl groups can be used, and examples of such mixed acyl groups include cocoyl groups. A representative content ratio for each of the acyl groups contained in the cocoyl group is C8:C10:C12:C14:C16:C18 = 5:6:55:17:10:7 (mass ratio).

In view of the preparation maintaining a viscosity suitable for use, the acyl group in component (A) is preferably a lauroyl group, a myristoyl group, an oleoyl group or a cocoyl group, and particularly preferably a lauroyl group, an oleoyl group or a cocoyl group. Furthermore, in view of not diminishing the moisture of the scalp following shampooing, even after further drying with a dryer immediately following towel drying, a lauroyl group and a cocoyl group are preferred, and a cocoyl group is particularly preferred.

In the formula, X is represented by H₃N⁺CH₂CH₂SO₃⁻M⁺, wherein M is an alkali metal or an organic ammonium. Specific examples of the M include alkali metals such as sodium and potassium, and organic ammoniums such as triethanolamine, arginine, lysine and histidine.

In view of the preparation maintaining a viscosity suitable for use, the M in the X in the general formula (1) may be any alkali metal or organic ammonium, but in view of not diminishing the moisture of the scalp following shampooing, even after further drying with a dryer immediately following towel drying, alkali metals such as sodium and potassium, or triethanolamine are preferred, and sodium and triethanolamine are particularly preferred.

One or more types of component (A) can be used in the hair shampoo composition of the present invention. Component (A) is included in the hair shampoo composition at 0.5 to 15 mass%, preferably 1 to 12 mass%, and particularly preferably 2 to 10 mass%. If the content is too high or too low, the preparation will not readily maintain a viscosity suitable for use, following shampooing, after further drying with a dryer immediately after towel drying, the moisture of the scalp may be diminished.

### Component (B)

Component (B) is an alkylene oxide derivative represented by the following general formula (2).

R²COO(BO)ₘH (2)

In the formula, R²CO is an acyl group having 8 to 14 carbon atoms, and specific examples of such an acyl group include a capryloyl group, a caproyl group, a lauroyl group, and a myristoyl group.

In view of the preparation maintaining a viscosity suitable for use, the acyl group in component (B) is preferably a caproyl group, a lauroyl group or a myristoyl group, and particularly preferably a lauroyl group or a myristoyl group. Furthermore, in view of not diminishing the moisture of the scalp after shampooing and drying, a lauroyl group and a myristoyl group are preferred, and a lauroyl group is particularly preferred.

In the formula, BO is an oxybutylene group, which is a group derived from 1,2-butylene oxide.

Further, m in the formula represents the average addition molar number of the oxybutylene group, and 1 ≤ m ≤ 2. If m in the formula has a numerical value within this range, the preparation will be able to maintain a viscosity suitable for use, and the moisture of the scalp will not readily be diminished after shampooing and drying.

One or more types of component (B) can be used in the hair shampoo composition of the present invention.

The component (B) is included in the hair shampoo composition at 0.3 to 10 mass%, preferably 0.5 to 7 mass%, and particularly preferably 1 to 5 mass%. If the content is too low, the preparation will not readily maintain a viscosity suitable for use, and following shampooing, after further drying with a dryer immediately after towel drying, the moisture of the scalp may be diminished. On the other hand, if the content is too high, the preparation will not readily maintain a viscosity suitable for use.

In the hair shampoo composition of the present invention, the mass ratio of component (A) to component (B) is (A)/(B) = 0.5 to 8, preferably 1 to 6, and particularly preferably 3 to 5. If the mass ratio (A)/(B) is too high, the preparation will not readily maintain a viscosity suitable for use, and following shampooing, after further drying with a dryer immediately following towel drying, the moisture of the scalp may be diminished. On the other hand, if the mass ratio (A)/(B) is too low, the preparation will not readily maintain a viscosity suitable for use.

An alkylene oxide derivative of component (C) represented by the following formula (3) can be further blended in the hair shampoo composition of the present invention, with the object of not diminishing the moisture of the scalp after shampooing and drying, and in particular with the object of not diminishing the moisture of the scalp after drying with a dryer.

R³COO(BO)ₙOCR⁴ (3)

In the formula, R³CO and R⁴CO each independently represent an acyl group having 8 to 14 carbon atoms, and specific examples of such an acyl group include a capryloyl group, a caproyl group, a lauroyl group and a myristoyl group. In view of the preparation maintaining a viscosity suitable for use, the acyl group in component (C) is preferably a caproyl group, a lauroyl group or a myristoyl group, and particularly preferably a lauroyl group or a myristoyl group. Furthermore, in view of not diminishing the moisture of the scalp after shampooing and drying, a lauroyl group and a myristoyl group are preferred, and a lauroyl group is particularly preferred.

In the formula, BO is an oxybutylene group, which is a group derived from 1,2-butylene oxide.

Further, n in the formula represents the average addition molar number of the oxybutylene group, and 1 ≤ n ≤ 2.

So as not to diminish the moisture of the scalp after drying with a dryer, the mass ratio of component (B) to component (C) is preferably (B)/(C) = 8 to 100, particularly preferably 10 to 75, and more preferably 20 to 50.

Note that, the content of component (C) in the hair shampoo composition is preferably 0.01 to 1.5 mass%, and particularly preferably 0.02 to 1 mass%.

One or more types of component (C) can be used in the hair shampoo composition of the present invention.

Furthermore, in view of the preparation maintaining a viscosity suitable for use and maintaining the moisture of the scalp after further drying with a dryer immediately after towel drying, the relationship between components (A) and (B) and component (C) is preferably {(A)/(B)} × (C) = 0.1 to 0.8, particularly preferably 0.1 to 0.5, and more preferably 0.15 to 0.3.

Amphoteric surfactants, cationized polymers, pearlizing agents, preservatives, chelating agents, perfumes and the like, which are components normally used in hair shampoos, can further be blended in the hair shampoo composition of the present invention. For example, coconut oil fatty acid amidopropyl betaine, lauryl betaine and the like are used as amphoteric surfactants, and the content thereof is usually 1 to 20 mass% of the hair shampoo composition. Cationized cellulose, cationized guar gum and the like are used as the cationized polymers, and the content thereof is usually 0.03 to 3 mass% of the hair shampoo composition. Further, ethylene glycol distearate or the like can suitably be blended as a pearlizing agent, methylparaben, phenoxyethanol or the like can suitably be blended as a preservative, and disodium ethylenediaminetetraacetate, citric acid and the like can suitably be blended as a chelating agent.

The hair shampoo composition of the present invention can be produced by conventional methods. Furthermore, the form of the preparation may be freely chosen, and transparent liquids, pearlized liquids, pastes, creams, gels and the like can be used.

### [Examples]

Hereafter, the present invention will be described in more detail with reference to examples and comparative examples.

### Examples 1 to 15 and Comparative Examples 1 to 12

The hair shampoo compositions shown in Tables 1 and 2 were prepared according to conventional methods. Evaluations of the ease of use (viscosity) of the preparation, the perceived moisture of the scalp after thorough rinsing of the hair shampoo composition and towel drying, and the perceived moisture of the scalp after further drying with a dryer were each performed by the following methods. The results are shown in Tables 1 and 2. Numerical values in the tables indicate mass% of pure content in the hair shampoo composition.

Note that, the m in the component (B) oxybutylene laurate indicates the average addition molar number of oxybutylene groups, and the n in the component (C) oxybutylene dilaurate indicates the average addition molar number of oxybutylene groups.

### A. Ease of use (viscosity) of the preparation

The hair shampoo composition was taken up in a hand, in the amount of 5 g, and the manner in which the hair shampoo composition remained in the hand was observed for 10 seconds. The ease of use of the hair shampoo composition at this time was evaluated according to the following three-level criteria.

### Ease of use of the preparation

### Score: Evaluation

2: Almost all of the preparation remains in hand, such that it is easy to use.
1: Some of the preparation runs out from the hand.
0: If the hand is not slightly cupped, most of the preparation will run out from the hand.

### B. Perceived moisture of the scalp after towel drying

The hair and scalp were thoroughly wetted with warm water at 40°C, the hair and scalp were shampooed for 30 seconds with 5 g of the hair shampoo composition, and then the hair and scalp were rinsed with warm water at 40°C for two minutes. The water on the hair and scalp was thoroughly removed by towel drying, and the perceived moisture of the scalp after 10 minutes was evaluated at the following four levels.

### Moisture of the scalp after towel drying

### Score: Evaluation

3: Feels moist.
2: Feels a little moist.
1: Feels somewhat lacking in moisture.
0: Feels lacking in moisture.

### C. Perceived moisture of the scalp after dryer drying

The hair and scalp were thoroughly wetted with warm water at 40°C, the hair and scalp were shampooed for 30 seconds with 5 g of the hair shampoo composition, and then the hair and scalp were rinsed with warm water at 40°C for two minutes. The water on the hair and scalp was thoroughly removed by towel drying and then drying with a dryer, and the perceived moisture of the scalp after 60 minutes was evaluated at the following four levels.

### Perceived moisture of the scalp after dryer drying

### Score: Evaluation

3: Feels moist.
2: Feels a little moist.
1: Feels somewhat lacking in moisture.
0: Feels lacking in moisture.

The evaluations A, B and C described above were performed by 20 expert panelists, and the average score was found for each. For evaluation A, an average score of 1.5 points or more was considered to be good, and for evaluations B and C, an average score of 2.0 points or more was considered to be good.

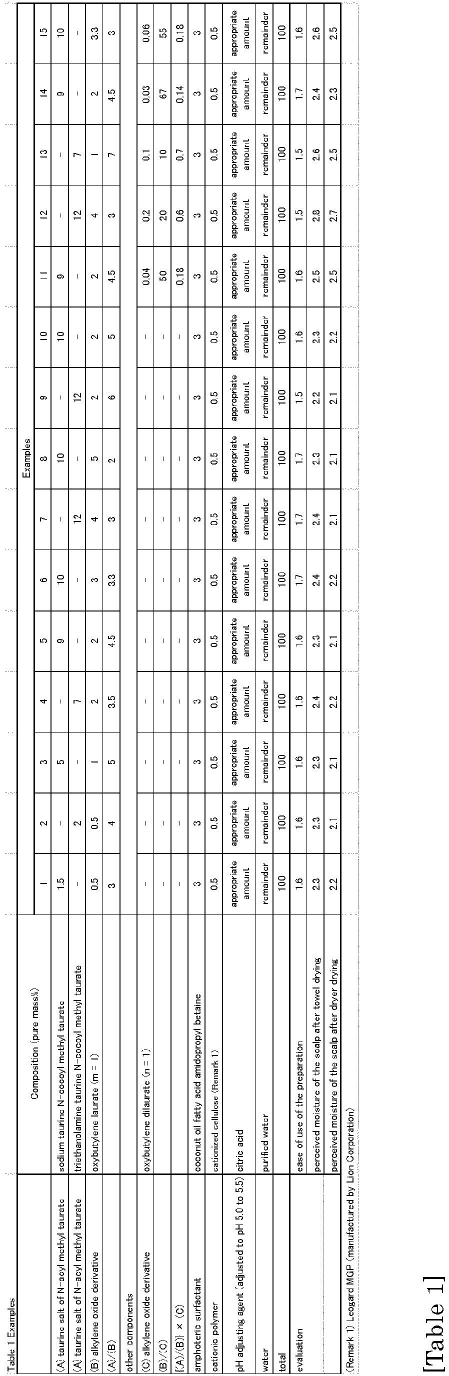

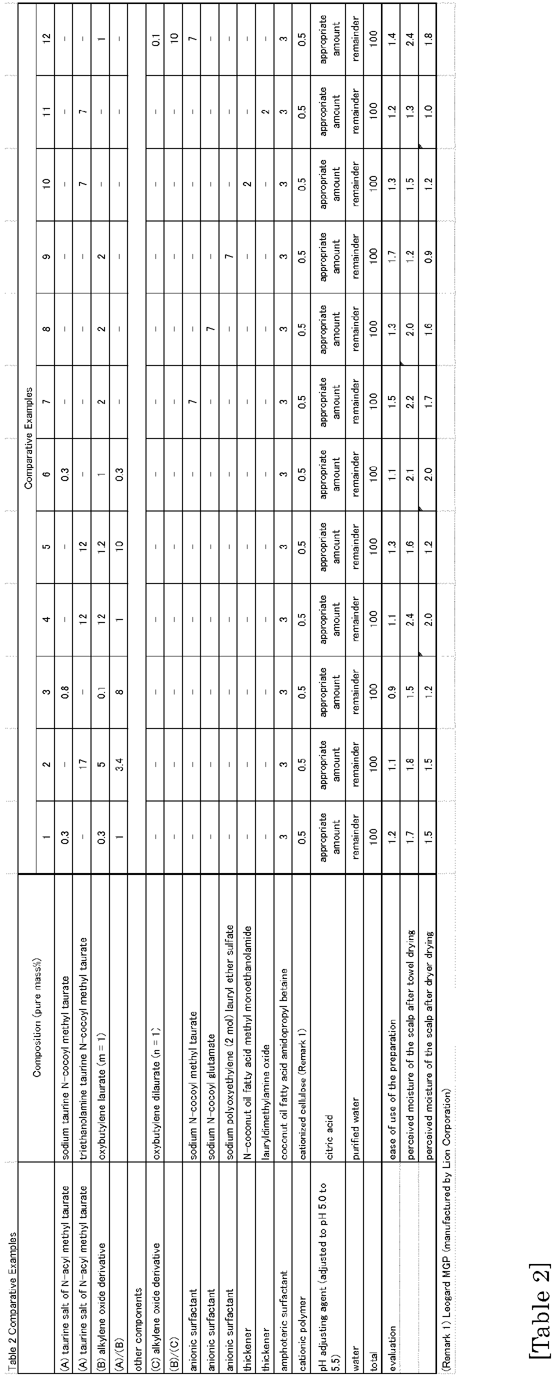

According to the evaluation results for Examples 1 to 15, all of the samples of the hair shampoo composition of the present invention were compositions with which the preparation retained a viscosity suitable for use, the moisture of the scalp was not diminished after shampooing and towel drying, and the moisture of the scalp was not diminished, even after further drying with a dryer.

On the other hand, in Comparative Examples 1 to 12, satisfactory effects are not obtained.

In Comparative Example 1, the content of the component (A) taurine salt of an N-acyl methyl taurate is less than 0.5 mass%, such that the viscosity of the preparation is insufficient and it is difficult to use, and both after towel drying and after dryer drying, the moisture of the scalp is diminished.

In Comparative Example 2, the content of the component (A) taurine salt of an N-acyl methyl taurate exceeds 15 mass%, such that the viscosity of the preparation is insufficient and it is difficult to use, and both after towel drying and dryer drying, the moisture of the scalp is diminished.

In Comparative Example 3, the content of the component (B) alkylene oxide derivative is less than 0.3 mass%, such that the viscosity of the preparation is insufficient and it is difficult to use, and both after the towel drying and after dryer drying, the moisture of the scalp is diminished.

In Comparative Example 4, the content of the component (B) alkylene oxide derivative exceeds 10 mass%, such that the viscosity of the preparation is insufficient and it is difficult to use.

In Comparative Example 5, the mass ratio (A)/(B) of the component (A) taurine salt of an N-acyl methyl taurate to the component (B) alkylene oxide derivative is greater than 8, such that the viscosity of the preparation is insufficient and it is difficult to use, and both after the towel drying and after dryer drying, the moisture of the scalp is diminished.

In Comparative Example 6, the mass ratio (A)/(B) of the component (A) taurine salt of an N-acyl methyl taurate to the component (B) alkylene oxide derivative is less than 0.5, such that the viscosity of the preparation is insufficient and it is difficult to use.

In Comparative Example 7, instead of the component (A) taurine salt of an N-acyl methyl taurate, sodium N-cocoyl methyl taurate is used as an anionic surfactant, such that the moisture of the scalp is diminished after dryer drying.

In Comparative Example 8, instead of the component (A) taurine salt of an N-acyl methyl taurate, sodium N-cocoyl glutamate is used as an anionic surfactant, such that the viscosity of the preparation is insufficient and it is difficult to use, and the moisture of the scalp is diminished after dryer drying.

In Comparative Example 9, instead of the component (A) taurine salt of an N-acyl methyl taurate, sodium polyoxyethylene (2 mol) lauryl ether sulfate is used as an anionic surfactant, such that both after towel drying and after dryer drying, the moisture of the scalp is diminished.

In Comparative Example 10, instead of the component (B) alkylene oxide derivative, N-coconut oil fatty acid methyl monoethanolamide is used as the thickener, such that the viscosity of the preparation is insufficient and it is difficult to use, and both after the towel drying and after dryer drying, the moisture of the scalp is diminished.

In Comparative Example 11, instead of the component (B) alkylene oxide derivative, lauryldimethylamine oxide is used as the thickener, such that the viscosity of the preparation is insufficient and it is difficult to use, and both after the towel drying and after dryer drying, the moisture of the scalp is diminished.

In Comparative Example 12, instead of the component (A) taurine salt of an N-acyl methyl taurate of Example 12, in which the component (C) alkylene oxide derivative is blended, sodium N-cocoyl methyl taurate is used as an anionic surfactant, such that the viscosity of the preparation is insufficient and it is difficult to use, and the moisture of the scalp is diminished after dryer drying.

### Formulation Example 1

A pearl hair shampoo composition, which was composed as shown in the following Table 3, was prepared by conventional methods. Mass% is the pure value. Note that, the m in the component (B) oxybutylene laurate indicates the average addition molar number of oxybutylene groups.

**[Table 3]**

| (component) | (mass%) |
|---|---|
| (A) sodium taurine N-cocoyl methyl taurate | 8.0 |
| (B) oxybutylene laurate (m = 1) | 2.0 |
| (B) oxybutylene laurate (m = 2) | 1.0 |
| ethylene glycol distearate | 2.0 |
| coconut oil fatty acid amidopropyl betaine | 3.0 |
| cationized guar gum (Remark 2) | 0.5 |
| sodium benzoate | 0.3 |
| phenoxyethanol | 0.5 |
| perfume | 0.5 |
| disodium ethylenediaminetetraacetate | 0.3 |
| citric acid | appropriate amount (adjusted to pH 5.0 to 5.5) |
| purified water | remainder |
| total | 100.0 |

| | |
|---|---|
| (Remark 2) Jaguar C (manufactured by Rhodia) | |

This composition was evaluated according to evaluation methods A, B and C, described above. The results are shown below.

### Evaluation Items: Average scores from 20 people

Ease of use (viscosity) of the preparation: 1.7
Perceived moisture of the scalp after towel drying: 2.6
Perceived moisture of the scalp after dryer drying: 2.5

According to the foregoing results, the pearl hair shampoo composition of Formulation Example 1 was a composition with which the preparation maintained a viscosity suitable for use, and did not diminish the moisture of the scalp following shampooing, even after further drying with a dryer immediately following towel drying.

### Formulation Example 2

A transparent hair shampoo composition, which was composed as shown in the following Table 4, was prepared by conventional methods. Mass% is the pure value. Note that, the m in the component (B) oxybutylene laurate indicates the average addition molar number of oxybutylene groups.

**[Table 4]**

| (component) | (mass%) |
|---|---|
| (A) sodium taurine N-cocoyl methyl taurate | 7.0 |
| (B) oxybutylene laurate (m = 1) | 2.5 |
| coconut oil fatty acid amidopropyl betaine | 2.0 |
| lauryl betaine | 1.0 |
| cationized cellulose (Remark 1) | 0.5 |
| dipropylene glycol | 1.5 |
| methylparaben | 0.2 |
| phenoxyethanol | 0.5 |
| perfume | 0.5 |
| disodium ethylenediaminetetraacetate | 0.2 |
| citric acid | appropriate amount (adjusted to pH 5.0 to 5.5) |
| purified water | remainder |
| total | 100.0 |

| | |
|---|---|
| (Remark 1) Leogard MGP (manufactured by Lion Corporation) | |

This composition was evaluated according to evaluation methods A, B and C, described above. The results are shown below.

### Evaluation Items: Average scores from 20 people

Ease of use (viscosity) of the preparation: 1.7
Perceived moisture of the scalp after towel drying: 2.6
Perceived moisture of the scalp after dryer drying: 2.5

According to the foregoing results, the transparent hair shampoo composition of Formulation Example 2 was a composition with which the preparation maintained a viscosity suitable for use, and did not diminish the moisture of the scalp following shampooing, even after further drying with a dryer immediately following towel drying.

### Formulation Example 3

A transparent hair shampoo composition, which was composed as shown in the following Table 5, was prepared by conventional methods. Mass% is the pure value.

**[Table 5]**

| (component) | (mass%) |
|---|---|
| (A) sodium taurine N-cocoyl methyl taurate | 7.0 |
| (B) oxybutylene laurate (m = 1) | 2.0 |
| (C) oxybutylene dilaurate (n = 1) | 0.04 |
| coconut oil fatty acid amidopropyl betaine | 8.0 |
| cationized cellulose (Remark 1) | 1.5 |
| dipropylene glycol | 1.5 |
| methylparaben | 0.2 |
| phenoxyethanol | 0.5 |
| perfume | 0.5 |
| disodium ethylenediaminetetraacetate | 0.2 |
| citric acid | appropriate amount (adjusted to pH 5.0 to 5.5) |
| purified water | remainder |
| total | 100.0 |

| | |
|---|---|
| (Remark 1) Leogard MGP (manufactured by Lion Corporation) | |

This composition was evaluated according to evaluation methods A, B and C, described above. The results are shown below.

### Evaluation Items: Average scores from 20 people

Ease of use (viscosity) of the preparation: 1.9
Perceived moisture of the scalp after towel drying: 2.5
Perceived moisture of the scalp after dryer drying: 2.5

According to the foregoing results, the transparent hair shampoo composition of Formulation Example 3 was a composition with which the preparation maintained a viscosity suitable for use, and did not diminish the moisture of the scalp following shampooing, even after further drying with a dryer immediately following towel drying.

## Claims

1. A hair shampoo composition comprising the following components (A) and (B):
(A) a taurine salt of an N-acyl methyl taurate represented by the following general formula (1) at 0.5 to 15 mass%
R¹CON(CH₃)CH₂CH₂SO₃⁻X⁺ (1)
(wherein R¹CO is an acyl group having 8 to 24 carbon atoms, X is H₃N⁺CH₂CH₂SO₃⁻M⁺, and M is an alkali metal or organic ammonium)
(B) an alkylene oxide derivative represented by the following general formula (2) at 0.3 to 10 mass%
R²COO(BO)ₘH (2)
(wherein R²CO is an acyl group having 8 to 14 carbon atoms, BO is an oxybutylene group, m is the average addition molar number, and 1 ≤ m ≤ 2), wherein the mass ratio of component (A) to component (B) is (A)/(B) = 0.5 to 8.

2. The hair shampoo composition according to claim 1, further comprising component (C):
(C) an alkylene oxide derivative represented by the following general formula (3)
R³COO(BO)ₙOCR⁴ (3)
(wherein R³CO and R⁴CO are each independently an acyl group having 8 to 14 carbon atoms, BO is an oxybutylene group, n is the average addition molar number, and 1 ≤ n ≤ 2).

## Patentansprüche

1. Haarshampoo-Zusammensetzung, aufweisend die folgenden Komponenten (A) und (B):
(A) ein Taurinsalz eines N-Acylmethyltaurats repräsentiert durch die folgende allgemeine Formel (1) bei 0,5 bis 15 Massen-%
R¹CON(CH₃)CH₂CH₂SO₃⁻X⁺ (1)
(wobei R¹CO eine Acylgruppe mit 8 bis 24 Kohlenstoffatomen ist, X H₃N⁺CH₂CH₂SO₃⁻M⁺ ist, und M ein Alkalimetall oder organisches Ammonium ist)
(B) ein Alkylenoxidderivat repräsentiert durch die folgende allgemeine Formel (2) bei 0,3 bis 10 Massen-%
R²COO(BO)ₘH (2)
(wobei R²CO eine Acylgruppe mit 8 bis 14 Kohlenstoffatomen ist, BO eine Oxybutylengruppe ist, m die durchschnittliche Additionsmolzahl ist, und 1 ≤ m ≤ 2), wobei das Massenverhältnis von Komponente (A) zu Komponente (B) ist (A)/(B) = 0,5 bis 8.

2. Haarshampoo-Zusammensetzung nach Anspruch 1, weiterhin aufweisend die Komponente (C):
(C) ein Alkylenoxidderivat repräsentiert durch die folgende allgemeine Formel (3)
R³COO(BO)ₙOCR⁴ (3)
(wobei R³CO und R⁴CO jeweils unabhängig voneinander eine Acylgruppe mit 8 bis 14 Kohlenstoffatomen sind, BO eine Oxybutylengruppe ist, n die durchschnittliche Additionsmolzahl ist, und 1 ≤ n ≤ 2).

## Revendications

1. Composition de shampooing pour les cheveux comprenant les composants suivants (A) et (B) :
(A) un sel de taurine d'un N-acyl méthyl taurate représenté par la formule générale suivante (1) à 0,5 à 15 % en masse
R¹CON(CH₃)CH₂CH₂SO₃⁻X⁺ (1)
(dans laquelle R¹CO est un groupe acyle ayant 8 à 24 atomes de carbone, X est H₃N⁺CH₂CH₂SO₃⁻M⁺ et M est un métal alcalin ou un ammonium organique)
(B) un dérivé d'oxyde d'alkylène représenté par la formule générale suivante (2) à 0,3 à 10 % en masse
R²COO(BO)ₘH (2)
(dans laquelle R²CO est un groupe acyle ayant 8 à 14 atomes de carbone, BO est un groupe oxybutylène, m est le nombre molaire d'addition moyen ,et 1 ≤ m ≤ 2),
dans laquelle le rapport massique du composant (A) au composant (B) est (A)/(B) = 0,5 à 8.

2. Composition de shampooing pour les cheveux selon la revendication 1, comprenant en outre le composant (C) :
(C) un dérivé d'oxyde d'alkylène représenté par la formule générale suivante (3) :
R³COO(BO)ₙOCR⁴ (3)
(dans laquelle R³CO et R⁴CO sont chacun indépendamment un groupe acyle ayant 8 à 14 atomes de carbone, BO est un groupe oxybutylène, n est le nombre molaire d'addition moyen, et 1 ≤ n ≤ 2).
